# EUROPEAN PATENT APPLICATION

(11) **EP 3 650 044 A1**
(43) Date of publication of application: **13.05.2020**
(21) Application number: 18204509.6
(22) Date of filing: 06.11.2018
(51) Int. Cl.: A61K 39/12, C07K 14/005

(54) **ANTI-GLYCAN VACCINES**

(71) Applicant: ETH Zürich, 8092 Zürich (CH); University of Bern, 3012 Bern (CH)
(72) Inventor: Aebi, Markus, 5430 Wettingen (CH); Wetter, Michael, 8047 Zurich (CH); Bachmann, Martin, 8487 Raemismuehle (CH); Keys, Timothy Gerard, 8307 Effretikon (CH); Tytgat, Hanne Lore, 8052 Zurich (CH)
(74) Representative: Kasche, André

(57) **Abstract**

**Summary**

The present invention is directed to a self-assembling glycosylated capsid polypeptide, wherein the capsid polypeptide comprises at least one 3-deoxy-D-*manno*-oct-2-ulosonic acid (Kdo)-glycosylation. The present invention is also directed to a method for producing the self-assembling glycosylated capsid polypeptide, related compositions comprising the glycosylated capsid polypeptide, as well as to related medical uses.

## Description

The present invention is directed to a self-assembling glycosylated capsid polypeptide, wherein the capsid polypeptide comprises at least one 3-deoxy-D-*manno*-oct-2-ulosonic acid (Kdo)-glycosylation. The present invention is also directed to a method for producing the self-assembling glycosylated capsid polypeptide, related compositions comprising the glycosylated capsid polypeptide, as well as to related medical uses.

### Background

The surface of all living cells, including bacterial pathogens, present a dense array of carbohydrate (glycan) structures. In principle, these surface glycans are the ideal vaccine antigen as they are the first structure encountered by the immune system. However, isolated glycans are poorly immunogenic, especially in children and the elderly - two populations in greatest need of the protection offered by vaccination. The solution to this problem is to covalently cross-link the carbohydrate antigen to an immunogenic carrier protein, generating a so-called glyco-conjugate vaccine. Broadly defined, there are two methods for the production of glycoconjugate vaccines.

The first production method is based on a highly complex multi-step process which involves separate production of the protein and glycan components followed by chemical conjugation (reviewed by Adamo et al., Chem Sci, 2013, 4: 2995-3008). This method is expensive, technically complex, and not useful for all glycans due to undesired modifications of the sugar structure during the coupling reactions.

The second production method is a biotechnological route where genetically engineered *Escherichia coli* produce and conjugate the protein and glycan components in the periplasmic space of the bacterium (i.e. bioconjugation; reviewed by Keys and Aebi, Curr Opin Syst Biol, 2017, 5: 23-31). This method exploits PglB, a periplasmic enzyme capable of transferring an entire oligosaccharide (glycan antigen) from the appropriate lipid-linked precursor onto a target protein. For this method the yield of glycoconjugate is low due to the requirement for periplasmic expression of the target protein, and it is only applicable for a small subset of glycan antigens, namely, those where the appropriate lipid-linked oligosaccharide precursor can be produced *in vivo.*

Traditionally, glycans are chemically conjugated to a toxoid, e.g. cholera or tetanus toxin, or *Pseudomonas aeruginosa* exotoxin in the case of the PglB system. Using toxoids as carriers presents two drawbacks. Firstly, the valency of glycan modification is limited to the number of reactive groups on the toxoid surface. Low sugar valency can lead to a predominant immune reaction against the protein carrier rather than the target sugar. Secondly, a limited number of toxoid carriers are already used for a large number of glycoconjugate vaccines resulting in variable immune interference (reviewed by Bröcker et al., Vaccine, 2017: 3286-3294).

It is the **objective technical problem** of the present invention to provide improved anti-glycan vaccines that can be prepared more easily and economically.

The problem is solved in a first aspect by the provision of a self-assembling glycosylated capsid polypeptide, wherein the capsid polypeptide comprises at least one 3-deoxy-D-*manno-*oct-2-ulosonic acid (Kdo)-glycosylation.

In contrast to chemical conjugation, the glycosylated capsid polypeptide of the present invention can be produced entirely *in vivo* in bacterial cytoplasm, and no further chemical modification or processing steps are necessary. The finished polypeptide can be extracted directly from the bacterial cells and purified for use. As opposed to the PglB-based system, the polypeptide of the present invention can be produced in a cytoplasmic glycosylation system that allows very high yields of glycoconjugates. Furthermore, no lipid-linked precursors are required, thereby providing access to a range of glycan structures that are not accessible to the PglB system.

The nanoscale size and multimeric properties of self-assembling capsid polypeptides make them highly immunogenic scaffolds for antigen presentation, which can, e.g., elicit strong serum and mucosal immunity.

For example, the glycosylated capsid polypeptides of the present invention can be produced in one step in the cytoplasm of a suitable host cell in high yields, with versatile glycosylation patterns and without the requirement for chemical modifications or lipid-based precursors.

It was surprisingly found that the presence of a 3-deoxy-D-*manno*-oct-2-ulosonic acid-(Kdo)-glycosyl residue is particularly advantageous because, e.g., this residue serves as an efficient antigen triggering an immune response in an organism, or the residue can function as a highly versatile starting block for biosynthetically attaching further glycosyl residues such as, e.g., further sugar molecules or capsular polysaccharides (CPS).

The self-assembling capsid polypeptides for use in the present invention encompass any polypeptides that are capable of self-assembly, i.e. polypeptides that form dimeric, trimeric or multimeric polypeptide structures by self-assembly. The number of polypeptides that assemble is not limited and can be any number greater than two, optionally greater than any of 5, 10, 11, 20 or even greater than 50 or 100. Optionally, the number of polypeptides that assemble is in the range from about 12 to about 360, optionally is about 180 polypeptide units. Optionally, the self-assembling capsid polypeptides for use in the present invention encompass polypeptides of the shell of a virus. The capsid polypeptides can be, e.g., the oligomeric structural subunits that make up the virus shell that encloses the genetic material of the virus. The capsid polypeptides, as used herein, are self-assembling, which means that they spontaneously assemble to form a three-dimensional di-, tri- or multimer, e.g. a capsid which is also known as a virus-like particle (VLP). The assembled capsid polypeptides (e.g. in the form of a capsid) can have any shape, geometry or structure, e.g. icosahedral or helical or planar. The assembled capsid polypeptides, e.g. the capsids or virus-like particles (VLPs), are highly immunogenic and make it possible to induce high levels of high-affinity and neutralizing antibodies without the use of adjuvants.

All aspects of the present invention, including products, methods and medical uses, are directed to the self-assembling glycosylated capsid polypeptide as well as to an assembly of multiple glycosylated capsid polypeptides resulting from self-assembly of at least two glycosylated capsid polypeptides.

The term "polypeptide", as used herein, is meant to comprise peptides, polypeptides, oligopeptides and proteins that comprise two or more amino acids linked covalently through peptide bonds. The term does not refer to a specific length of the product. Polypeptides include post-translational modifications of the polypeptides, for example, glycosylations, acetylations, phosphorylations, cleavages and the like. The term polypeptide also encompasses polypeptide analogs, polypeptides comprising non-natural amino acids, peptidomimetics, β-amino acids, etc.

The term "polypeptide", as used herein, also encompasses an isolated and purified glycosylated or non-glycosylated polypeptide. The term "isolated and purified polypeptide", as used herein, refers to a glycosylated or non-glycosylated polypeptide or a peptide fragment which either has no naturally-occurring counterpart (e.g., a peptidomimetic), or has been separated and/or purified from components which naturally accompany it. Optionally, a polypeptide is considered "isolated and purified" when it makes up for at least 40% (w/w) in non-glycosylated and/or at least 60 % (w/w) in glycosylated form, of a dry preparation, thus being free from most naturally-occurring polypeptides and/or organic molecules with which it is naturally associated. Optionally, a polypeptide or glycosylated polypeptide for use in the invention makes up for at least 80%, optionally at 90%, and optionally at least 99% (w/w) of a dry preparation. Optionally, glycosylated or non-glycosylated polypeptides for use in the invention make up for at least at least 80%, optionally at least 90%, and optionally at least 99% (w/w) of a dry polypeptide preparation.

Furthermore, the present invention also encompasses crude extracts, e.g. for use as vaccines, wherein the glycosylated polypeptide makes up for at least about 5%, 10% or 15% of the dry crude preparation.

The self-assembling capsid polypeptides of the present invention are glycosylated. The glycosylation can be at any amino acid of the capsid polypeptide, optionally at a glycosylation site on the outer surface of the assembled capsid. Optionally, the self-assembling capsid polypeptides comprise a suitable glycosylation site which can be naturally occurring or which can be introduced artificially, e.g. by fusing a suitable glycosylation site sequence into the capsid polypeptide sequence. The term "glycosylated", as used herein, refers to any sugar or carbohydrate residue attached to the polypeptide structure, e.g. mono-, di-, tri- or polysaccharide structures which can be linked by any glycosidic bond, e.g. by α- or β-glycosidic bonds.

The 3-deoxy-D-*manno*-oct-2-ulosonic acid-(Kdo) glycosyl residue of the polypeptide of the present invention can be linked to the polypeptide either directly or via other sugar residues, and the Kdo can be linked to the polypeptide at a nitrogen or oxygen atom of the polypeptide. Also, the glycosylated capsid polypeptide of the present invention can comprise further sugars or polysaccharides in addition to the Kdo glycosyl residue.

In another embodiment, the capsid polypeptide of the present invention is one, wherein the capsid polypeptide comprises a glycosylation pattern selected from the group consisting of
(1) βKdo-(2,3)αRha-(1,3)-βGlc-(1,N)- or βKdo-(2,3)-αRha-(1,3)-βGlcNAc-(1,O)-;
(2) αKdo-(2,6)-βGal-(1,4)-βGlc-(1,N)- or αKdo-(2,6)-βGal-(1,4)-βGlcNAc-(1,O)-;
(3) [βKdo-(2,4)]ₘ-[βKdo-(2,7)-βKdo-(2,4)]ₙ-βKdo-(2,3)-αRha-(1,3)-βGlc-(1,N)- or [βKdo-(2,4)]ₘ-[βKdo-(2,7)-βKdo-(2,4)],-βKdo-(2,3)-αRha-(1,3)-βGlcNAc-(1,O)-;
(4) [βKdo-(2,4)]ₘ-[βKdo-(2,7)-βKdo-(2,4)]ₙ-αKdo-(2,6)-βGal-(1,4)-βGlc-(1,N)- or [βKdo-(2,4)]ₘ-[βKdo-(2,7)-βKdo-(2,4)]ₙ-αKdo-(2,6)-βGal-(1,4)-βGlcNAc-(1,O)-;
(5) [βKdo-(2,7)]ₘ-[βKdo-(2,4)-βKdo-(2,7)]ₙ-βKdo-(2,3)-αRha-(1,3)-βGlc-(1,N)- or βKdo-(2,7)]ₘ-[βKdo-(2,4)-βKdo-(2,7)]ₙ-βKdo-(2,3)-αRha-(1,3)-βGlcNAc-(1,O)-; and
(6) [βKdo-(2,7)]ₘ-[βKdo-(2,4)-βKdo-(2,7)]ₙ-αKdo-(2,6)-βGal-(1,4)-βGlc-(1,N)- or [βKdo-(2,7)]ₘ-[βKdo-(2,4)-βKdo-(2,7)]ₙ-αKdo-(2,6)-βGal-(1,4)-βGlcNAc-(1,O)-;
wherein m is 0 or 1 and n is an integer from 2 to 4 and wherein the glycosylation pattern is connected to the capsid polypeptide at the reducing end.

A glycosylation pattern described, e.g., as "βKdo-(2,3)-αRha-(1,3)-βGlc-(1,N)-" indicates that the glycosyl residue is attached via the βGlc-unit (the reducing end) to a nitrogen of the polypeptide, whereas a glycosylation pattern described, e.g., as "βKdo-(2,3)-αRha-(1,3)-βGlcNAc-(1,O)-" indicates that the glycosyl residue is attached via the βGlcNAc-unit (the reducing end) to an oxygen of the polypeptide. One or more of the glycosylation pattern(s) can be attached to any amino acid residue(s) suitable for accepting the glycosyl residue(s), optionally via the oxygen of Ser and/or Thr or via the nitrogen of Asn.

Without wishing to be bound by theory, it is believed that the optional glycosylation patterns for use in the present invention represent (1) highly efficient antigens for triggering an immune response and (2) primer sites for further glycosylation enzymes, such as, e.g., enzymes that attach and thereby extend the glycosylation pattern by further sugar(s), e.g. capsular polysaccharides (CPS).

In another embodiment, the glycosylated capsid polypeptide of the present invention is one, wherein the at least one glycosylation is an N- or O-glycosylation of the capsid polypeptide, optionally
(1) an N-glycosylation at a glycosylation site Asn-X-Ser/Thr of the capsid polypeptide, wherein X is not Pro, or
(2) an O-glycosylation at a glycosylation site M-N-O-P, wherein
   M is selected from the group consisting of Pro, Thr and Arg,
   N is selected from the group consisting of Val, Thr and Arg,
   O is selected from the group consisting of Ser and Thr, and
   P is selected from the group consisting of Arg, Leu, Val, Ser and Thr (SEQ ID NO: 136).

Without wishing to be bound by theory, it is believed that the O-glycosylation takes place at the Ser/Thr part of the glycosylation site M-N-O-P (SEQ ID NO: 136), while the flanking amino acids represent recognition sites for the glycosylating enzymes.

The N- or O-glycosylation site sequences described above can be naturally occurring in the capsid polypeptides or they can be artificially introduced by fusing these sequences to the polypeptide sequence. Optionally, these sequences are presented on the surface of the three-dimensional structure of the assembled capsid polypeptides, e.g. for better presentation of the glycosyl residue(s) as antigens.

In another embodiment, the capsid polypeptide of the present invention is one, wherein the glycosylation pattern is selected as described herein and extended by at least one further glycoside, optionally capsular polysaccharides (CPS), optionally a CPS selected from the group consisting of:
(a) *E. coli* CPS selected from the group consisting of CPS K1, K2a/K2b, K3 to K6, K7/K56, K10, K11, K12/82, K13 to K16, K18 to K20, K22 to K24, K51 to K54, K74, K92, K93, K95 to K98 and K100;
(b) *Neisseria meningitidis* CPS selected from the group consisting of A to C, E, H, I, K, L, W, X, Y and Z;
(c) *Pasteurella multocida* CPS selected from the group consisting of CPS A, D and F;
(d) *Haemophilus influenzae* CPS selected from the group consisting of CPS a to d, e, e', and f;
(e) *Campylobacter jejuni* CPS selected from the group consisting of CPS HS1/44, HS2 to HS6, HS8 to HS12, HS15, HS18, HS19, HS21, HS22, HS23/36, HS27, HS29, HS32, HS33, HS35, HS37, HS38, HS40 to HS42, HS45, HS52, HS53, HS55, HS57, HS58, HS60, HS62, and HS63;
(f) *Actinobacillus pleuropneumoniae* CPS selected from the group consisting of CPS 1 to 18;
(g) *Bibersteinia trehalosi* CPS selected from the group consisting of CPS T3, T4 and T15;
(h) *Mannheimia haemolytica* CPS selected from the group consisting of A1 and A2; and
(i) *Cronobactersakazakii* CPS selected from the group consisting of K1 and K2.

The above-described CPS structures are known to the skilled person by the names indicated. For easier understanding, an overview of the CPS names and corresponding literature references is given in the overview of CPS names below.

For example, the CPS structure in an embodiment of the present invention is linked to the Kdo sugar at the non-reducing end of the glycosylation pattern. An exemplary *Neisseria meningitidis* group C CPS conjugate is
[-9)αNeu5Ac-(1]ₙ-[βKdo-(2,7)-βKdo-(2,4)]₂-βKdo-(2,3)-αRha-(1,3)-βGlc-(1,N)-, wherein n can be any integer from 1 to 200, optionally 10 to 100.

In another embodiment, the glycosylated capsid polypeptide of the present invention is one, wherein the self-assembling capsid polypeptide is selected from the group consisting of:
(a) a capsid polypeptide comprising or consisting of an amino acid sequence selected from the group consisting of SEQ ID NOs: 1 and 91 to 108;
(b) a capsid polypeptide comprising an amino acid sequence having an amino acid sequence identity of at least 70% or 80%, optionally at least 90% or 95%, optionally at least 98% with an amino acid sequence selected from the group consisting of SEQ ID NOs: 1 and 91 to 108; and
(c) a capsid polypeptide comprising a functional derivative and/or functional fragment of (a) and/or (b).

The percentage identity of any related amino acid molecules as described herein can be determined with the assistance of known methods. In general, special computer programs are employed that use algorithms adapted to accommodate the specific needs of this task. Exemplary methods for determining amino acid identity begin with the generation of the largest degree of identity among the sequences to be compared. Exemplary computer programs for determining the identity among two amino acid sequences comprise, but are not limited to, TBLASTN, BLASTP, BLASTX, TBLASTX (Altschul et al., J. Mol. Biol., 215, 403-410, 1990), or ClustalW (Larkin MA et al., Bioinformatics, 23, 2947-2948, 2007). The BLAST programs can be obtained from the National Center for Biotechnology Information (NCBI) and from other sources (BLAST handbook, Altschul et al., NCB NLM NIH Bethesda, MD 20894). The ClustalW program can be obtained from http://www.clustal.org.

The term "functional derivative" of a self-assembling capsid polypeptide for use in the present invention is meant to include any polypeptide or fragment thereof that has been chemically or genetically modified in its amino acid sequence, e.g. by addition, substitution and/or deletion of amino acid residue(s) and/or has been chemically modified in at least one of its atoms and/or functional chemical groups, e.g. by additions, deletions, rearrangement, oxidation, reduction, etc. as long as the derivative (1) has self-assembling properties, e.g. is capable of forming a di or multimer, e.g. capsid or partial capsid structure, (2) can be glycosylated, i.e. provides suitable amino acids or amino acid sequences as described herein, optionally comprising Ser, Thr and/or Asn, for glycosylation, and (3) optionally is immunogenic. The functional derivatives of self-assembling capsid polypeptides described herein are polypeptides that form dimeric, trimeric or multimeric polypeptide structures by self-assembly. The number of polypeptides that assemble is not limited and can be any number greater than two, optionally greater than eleven and optionally up to 360. An exemplary assay for determining the self-assembling properties of a given functional derivative is given below.

The self-assembling properties of a polypeptide can be determined by comparison of the size of the particle formed by the purified polypeptide in its "native" or folded state and after denaturation. If the radius of the particle formed by the folded polypeptide is greater than 10-fold that of the denatured polypeptide, the polypeptide is considered to self-assemble. For measuring the size of the particle in its folded state, the purified polypeptide can be equilibrated in phosphate buffered saline or any other common physiological buffer solution. For measuring the size of the particle in its denatured state, the purified polypeptide can be incubated for 30 minutes at 37 °C in a denaturing solution including, e.g., about 50 mM dithiothreitol and, e.g., about 8 M urea or, e.g., about 6 M guanidine hydrochloride. Exemplary methods for determining the size of particles formed by a given polypeptide include size exclusion chromatography, dynamic light scattering, and transmission electron microscopy.In this context, a "functional fragment" of a capsid polypeptide, as used herein, is one that forms part of a polypeptide or derivative of the invention and which maintains the same functional limitations as outlined above for the polypeptides and functional derivatives for use in the present invention.

In another embodiment, the capsid polypeptide of the present invention is one, wherein
(a) the self-assembling capsid polypeptide is a capsid polypeptide comprising an amino acid sequence according to SEQ ID NO: 1,
(b) the glycosylation pattern is [βKdo-(2,4)]ₘ-[βKdo-(2,7)-βKdo-(2,4)]ₙ-βKdo-(2,3)-αRha-(1,3)-βGlc-(1,N), wherein m is 0 or 1 and n is an integer from 2 to 4, optionally 2, and
(c) the at least one further glycoside is absent or is a CPS selected from the group consisting of: *Neisseria meningitidis* serogroup A, C, L, W-135, X, and Y; *Haemophilus influenzae* type b and c; *Pasteurella multocida* serogroup A; *Actinobacillus pleuropneumoniae* serotype 6 and 8; *Mannheimia haemolytica* serotype A1; *Escherichia coli* serotype K2, K16, K20, K22, K23 and K100; *Campylobacter jejuni* serotype HS1/44 HS2, HS3, HS4, HS5, HS8; and *Cronobacter sakazakii* serotype K1 and K2.

In another embodiment, the capsid polypeptide of the present invention is one, wherein
(a) the self-assembling capsid polypeptide is a capsid polypeptide comprising an amino acid sequence according to SEQ ID NO: 1,
(b) the glycosylation pattern is [βKdo-(2,7)]ₘ-[βKdo-(2,4)-βKdo-(2,7)]ₙ-βKdo-(2,3)-αRha-(1,3)-βGlc-(1,N), wherein m is 0 or 1 and n is an integer from 2 to 4, optionally 2, and
(c) the at least one further glycoside is absent or is a CPS selected from the group consisting of: *Neisseria meningitidis* serogroup A, C, L, W-135, X, and Y; *Haemophilus influenzae* type b and c; *Pasteurella multocida* serogroup A; *Actinobacillus pleuropneumoniae* serotype 6 and 8; *Mannheimia haemolytica* serotype A1; *Escherichia coli* serotype K2, K16, K20, K22, K23 and K100; *Campylobacter jejuni* serotype HS1/44 HS2, HS3, HS4, HS5, HS8; and *Cronobacter sakazakii* serotype K1 and K2.

In another aspect, the present invention is directed to a method for producing self-assembling capsid polypeptides as described herein, comprising the steps of:
(i) providing at least one bacterial host cell expressing in its cytoplasm
   (1) at least one self-assembling capsid polypeptide comprising at least one glycosylation site, optionally an N- or O-glycosylation site on the outer surface of the assembled capsid;
   (2) at least one N- or 0- glycosyl transferase enzyme (NGT or OGT) for glycosylating the at least one glycosylation site of the capsid polypeptide; and
   (3) at least one glycosylation enzyme for glycosylating the glycosyl residue(s) of the at least one glycosylation site of the capsid polypeptide of step (2),
(ii) culturing the host cell under physiological conditions so that
   (1) self-assembling capsid polypeptides are expressed, and
   (2) the at least one glycosyl transferase enzyme (NGT or OGT) glycosylates the at least one glycosylation site of the capsid polypeptide, and
   (3) the at least one glycosylation enzyme glycosylates the glycosyl residue(s) of the at least one glycosylation site of the capsid polypeptide;
   optionally isolating the glycosylated capsid polypeptide(s) from the host cell

All definitions given above also apply in the context of the method of the present invention, unless specifically noted otherwise.

The bacterial host cell for use in the present method expresses the self-assembling capsid polypeptide(s), the NGT or OGT and the at least one glycosylation enzyme for glycosylating the glycosyl residue(s) of the at least one glycosylation site of the capsid polypeptide in its cytoplasm. Therefore, the expression of the polypeptide and the glycosylation reactions can all take place in the cytoplasm which allows for an efficient production of the glycosylated polypeptides in high yields.

The at least one glycosyl transferase enzyme (NGT or OGT) can glycosylate one or more sites on the capsid polypeptide with one or more glycosyl residues, including mono-, di-, tri- or polysaccharides, optionally with Glc or GlcNAc, optionally with a single Glc or GlcNAc per glycosylation site if a Kdo residue is attached by the at least one glycosylation enzyme for glycosylating in step (i)(3) and (ii)(3) of the above-described method.

The at least one glycosylation enzyme of step (i)(3) and (ii)(3) for glycosylating the glycosyl residue(s) of the at least one glycosylation site of the capsid polypeptide introduces at least one further glycosyl residue at the glycosyl residue that was attached by the at least one glycosyl transferase enzyme (NGT or OGT) in step (i)(2) and (ii)(2). The glycosyl residue attached by the glycosylation enzyme of step (i)(3)/(ii)(3) can be any glycosyl residue, including mono-, di-, tri- or polysaccharides, as long as the glycosylated capsid polypeptide resulting from the method of the present invention features at least one Kdo glycosyl residue.

The host cells are cultured under physiological conditions which are conditions that allow for the expression of the polypeptides and the glycosylation reactions to take place, optionally also allow for growth and cultivation of the host cells. The self-assembly of the capsid polypeptides can occur inside or outside the host cell and before or after the glycosylation reactions.

The optional step of isolating the glycosylated capsid polypeptide(s) can yield an "isolated and purified polypeptide" as defined above.

In another embodiment, the method of the present invention features two or more bacterial host cells, each of them expressing at least one of (a) the capsid polypeptide, (b) the N- or 0- glycosyl transferase enzyme (NGT or OGT) and/or (c) the glycosylation enzyme for glycosylating the glycosyl residue(s) of the at least one glycosylation site of the capsid polypeptide.

In another embodiment, the method of the present invention is one, wherein the bacterial host cell(s) is a gram-negative or gram-positive host cell, optionally selected from the group consisting of *Escherichia* species, *Shigella* species, *Neisseria* species, *Haemophilus* species, *Pasteurella* species, *Actinobacillus* species, *Klebsiella* species, *Xhantomonas* species, *Salmonella* species, *Yersinia* species, *Lactococcus* species, *Lactobacillus* species, *Pseudomonas* species, *Corynebacterium* species, *Streptomyces* species, *Streptococcus* species, *Staphylococcus* species, *Bacillus* species, and *Clostridium* species.

In another embodiment, the method of the present invention is one, wherein the bacterial host cell(s) is (are) a cell-free system derived from a bacterial cell by lysis. E.g., lysis can be achieved mechanically or enzymatically.

In another embodiment, the method of the present invention is one, wherein the self-assembling capsid polypeptide is selected from the group consisting of:
(a) a capsid polypeptide comprising or consisting of an amino acid sequence selected from the group consisting of SEQ ID NO: 1 and 91 to 108;
(b) a capsid polypeptide comprising an amino acid sequence having an amino acid sequence identity of at least 70% or 80%, optionally at least 90% or 95%, optionally at least 98% with an amino acid sequence selected from the group consisting of SEQ ID NOs: 1, and 91 to 108; and
(c) a capsid polypeptide comprising a functional derivative and/or functional fragment of (a) and/or (b)

In another embodiment, the method of the present invention is one, wherein the at least one glycosyl transferase enzyme (NGT or OGT) is selected from the group consisting of:
(a) a glycosyl transferase enzyme comprising or consisting of an amino acid sequence selected from the group consisting of (SEQ ID NO: 2) and SEQ ID NOs: 109 to 133,
(b) a glycosyl transferase enzyme comprising an amino acid sequence having an amino acid sequence identity of at least 70% or 80%, optionally at least 90% or 95%, optionally at least 98% with an amino acid sequence selected from the group consisting of SEQ ID NOs: 2 and 109 to 133; and
(c) a glycosyl transferase enzyme comprising a functional derivative and/or functional fragment of (a) and/or (b).

In another embodiment, the method of the present invention is one, wherein the at least one glycosylation enzyme for glycosylating the glycosyl residue(s) of the at least one glycosylation site of the capsid polypeptide in steps (i)(3) and (ii)(3) as described above is selected from the group consisting of
(3a) a WbbB homolog comprising or consisting of an amino acid sequence selected from the group consisting of SEQ ID NOs: 3 and 29 to 90;
(3b) a bacterial polymerizing 3-deoxy-β-D-manno-oct-2-ulopyranosonic acid (β-Kdo) glycosyltransferase comprising or consisting of an amino acid sequence selected from the group consisting of SEQ ID NOs: 4 and 8 to 18;
(3c) an LgtB glycosylation enzyme comprising or consisting of an amino acid sequence according to SEQ ID NO: 5;
(3d) a Psp2,6ST homolog comprising or consisting of an amino acid sequence selected from the group consisting of SEQ ID NOs: 7 and 19 to 28; and
(3e) an alpha1,6GlcT homolog comprising or consisting of an amino acid sequence selected from the group consisting of SEQ ID NOs: 6, 134 and 135;
(3f) a glycosylation enzyme comprising an amino acid sequence having an amino acid sequence identity of at least 70% or 80%, optionally at least 90% or 95%, optionally at least 98% with an amino acid sequence selected from the group consisting of SEQ ID NOs: 3 to 90, 134 and 135; and
(3g) a glycosylation enzyme comprising a functional derivative and/or functional fragment of any of (3a) to (3f);
wherein the LgtB glycosylation enzyme of (3c), (3f) or (3g) or the alpha1,6GlcT homolog of (3e), (3f) or (3g) requires the presence of at least one further glycosylation enzyme selected from the WbbB homolog of (3a), (3f) or (3g), the bacterial polymerizing 3-deoxy-β-D-manno-oct-2-ulopy-ranosonic acid (β-Kdo) glycosyltransferase of (3b), (3f) or (3g) and/or the Psp2,6ST homolog of (3d), (3f) or (3g).

In the context of glycosyl transferase or glycosylation enzymes, the term "functional derivative" of a glycosyl transferase or glycosylation enzyme as described for the present invention is meant to include any glycosyl transferase or glycosylation enzyme or fragment thereof that has been chemically or genetically modified in its amino acid sequence, e.g. by addition, substitution and/or deletion of amino acid residue(s) and/or has been chemically modified in at least one of its atoms and/or functional chemical groups, e.g. by additions, deletions, rearrangement, oxidation, reduction, etc. as long as the derivative still has at least some glycosyl transferase or glycosylation activity to a measurable extent, e.g. of at least about 1 to 10%, optionally at least about 20 to 50%, optionally at least about 50 to 90% glycosyl transferase or glycosylation activity of the unmodified glycosyl transferase or glycosylation enzyme, e.g. a glycosyl transferase or glycosylation enzyme comprising an amino acid sequence according to SEQ ID NOs: 2 to 90, 109 to 133, 134 and 135 for use in the invention. An assay for determining glycosylase transferase or glycosylation enzyme activity is given below.

An exemplary method for determining if a glycosyltransferase, or a derivative thereof, is active, is to incubate the candidate enzyme together with a donor sugar (e.g. UDP-Glc, UDP-Gal, CMP-Kdo, or CMP-Neu5Ac, etc.) and an acceptor substrate (e.g. a fluorescently labelled oligosaccharide or glycopeptide) and determine if the acceptor substrate has been modified by reversed phase HPLC with fluorescence detection or mass spectrometry.

A second exemplary method is to co-express the candidate enzyme in *E. coli* together with an appropriate substrate protein or glycoprotein. Active derivatives will modify the substrate protein as determined by mass spectrometric analysis of the intact substrate protein. In this context a "functional fragment", as used herein, is one that forms part of a glycosylase transferase or glycosylation enzyme described herein and maintains the same functional limitations as outlined above for the functional derivatives.

In another embodiment, the method of the present invention is one, wherein the at least one bacterial host cell further expresses enzyme(s) or enzyme complex(es) for extending the glycosylation of the capsid polypeptide with at least one capsular polysaccharide (CPS), optionally a CPS selected from the group consisting of:
(a) *E. coli* CPS selected from the group consisting of CPS K1, K2a/K2b, K3 to K6, K7/K56, K10, K11, K12/82, K13 to K16, K18 to K20, K22 to K24, K51 to K54, K74, K92, K93, K95 to K98 and K100;
(b) *Neisseria meningitidis* CPS selected from the group consisting of A to C, E, H, I, K, L, W, X, Y and Z;
(c) *Pasteurella multocida* CPS selected from the group consisting of CPS A, D and F;
(d) *Haemophilus influenzae* CPS selected from the group consisting of CPS a to d, e, e', and f;
(e) *Campylobacter jejuni* CPS selected from the group consisting of CPS HS1/44, HS2 to HS6, HS8 to HS12, HS15, HS18, HS19, HS21, HS22, HS23/36, HS27, HS29, HS32, HS33, HS35, HS37, HS38, HS40 to HS42, HS45, HS52, HS53, HS55, HS57, HS58, HS60, HS62, and HS63;
(f) *Actinobacillus pleuropneumoniae* CPS selected from the group consisting of CPS 1 to 18;
(g) *Bibersteinia trehalosi* CPS selected from the group consisting of CPS T3, T4 and T15;
(h) *Mannheimia haemolytica* CPS selected from the group consisting of A1 and A2; and
(i) *Cronobacter sakazakii* CPS selected from the group consisting of K1 and K2;
optionally enzymes selected from the group consisting of serotype specific CPS biosynthesis enzymes.

In the above-noted organisms, the genetic machinery required for capsule biosynthesis is clustered at a single chromosomal locus known as the capsule gene cluster. These clusters have a conserved genetic organisation with two sets of genes, one set that are conserved across all of the listed capsule producing bacteria, and a second set of genes that are serotype specific (reviewed by Roberts, Ann Rev Microbiol, 1996, 50: 285-325). The serotype specific genes encode for the serotype specific CPS biosynthesis enzymes, which produce the serotype specific CPS.

In the following, the optional CPS for use in the present invention are additionally described by reference to the gene location(s). In the case of *E. coli* the serotype specific CPS biosynthesis genes are those in the region between *kpsMF* and *kpsFEDUCS* (Roberts, Ann Rev Microbiol, 1996, 50: 285-325). In the case of *N. meningitidis,* the serotype specific CPS biosynthesis genes are those in the region between *ctrABCD* and *galE* as defined in (Harrison et al., Emerg Infect Dis, 2013, 19 (4): 566-573). In the case of *P. multocida,* the serotype specific CPS biosynthesis genes are those in the region between *hexDCBA* and *phyAB* as defined in (Watt et al., FEMS Microbiol Lett, 2003, 225 (1): 9-14). In the case of *H. influenzae,* the serotype specific CPS biosynthesis genes are those in the genomic region between *bexDCAB* and *hcsAB* as defined in (Giufre et al, J Clin Microbiol, 2010, 48 (4): 1404-1407). In the case of *C. jejuni,* the serotype specific CPS biosynthesis genes are those in the region between *kpsF* and *kpsC* as defined in (Poly et al., PLOS ONE, 2015, 10 (12): e0144349). In the case of *A. pleuropneumoniae,* the serotype specific CPS biosynthesis genes are those in the genomic region between *cpsDCAB* and *ydeN* as defined in (Bossé et al., Vet Microbiol, 2018, 220 (July): 83-89). In the case of M. *haemolytica,* the serotype specific CPS biosynthesis genes are those in the genomic region between *wza* and *wbrA* as defined in (Lo et al., Infect Immun, 2001, 69 (7): 4458-4464). In the case of *C. sakazakii,* the serotype specific CPS biosynthesis genes are those in the genomic region between *kpsEDCS* and *kpsMT* as defined in (Ogrodzki and Forsythe, BMC Genomics, 2015, 16 (1)).

In another aspect, the present invention is directed to a composition, optionally a medical composition, comprising a self-assembling glycosylated capsid polypeptide of the present invention, optionally further comprising immunogenic compounds, optionally mRNA.

The optional mRNA can be any mRNA from any suitable source that, for example, is suitable for stimulating an immune response in an organism, optionally in a human or animal, optionally mammalian animal.

In another aspect, the present invention is directed to the self-assembling glycosylated capsid polypeptide described above or the composition described above for use in medical treatment, optionally for use as a vaccine.

For therapeutic use, the polypeptide or composition of the invention may be administered in any conventional dosage form in any conventional manner. Routes of administration include, oral, intravenous, intramuscular and subcutaneous injections. The modes of administration include but are not limited to oral, intramuscular, intradermal, intranasal or subcutaneous.

The polypeptide(s) or composition of the present invention may be administered alone or in combination with adjuvants that enhance stability of the compounds, facilitate administration of pharmaceutical compositions containing them in certain embodiments, provide increased dissolution or dispersion, increase activity, boost the immune response to produce more antibodies and longer-lasting immunity, thus minimizing the dose of antigen, polypeptide or composition needed (e.g. aluminum hydroxide, paraffin oil, killed bacteria, toxoids, squalene, IL-1, IL-2, IL-12, or Freud's complete or incomplete adjuvant), and the like, including other active ingredients. Advantageously such combination therapies utilize lower dosages of the conventional therapeutics, thus avoiding possible toxicity and adverse side effects incurred when those agents are used as monotherapies. The above-described polypeptide(s) or composition may be physically combined with conventional therapeutics or adjuvants into a single pharmaceutical composition. Reference in this regard is made to Cappola et al.: U.S. patent application no. 09/902,822, PCT/US 01/21860 und US provisional application no. 60/313,527, each incorporated by reference herein in their entirety. Advantageously, the polypeptide(s) or composition may then be administered together in a single dosage form. In some embodiments, the pharmaceutical compositions comprising such combinations of polypeptide(s) contain at least about 0.001%, optionally at least about 0.01% of a polypeptide of the present invention (w/w). The optimum percentage (w/w) of a polypeptide of the invention may vary and is within the purview of those skilled in the art. Alternatively, the polypeptides may be administered separately (either serially or in parallel). Separate dosing allows for greater flexibility in the dosing regime.

As mentioned above, dosage forms of the polypeptide(s) described herein include pharmaceutically acceptable carriers and adjuvants known to those of ordinary skill in the art. Methods for preparing such dosage forms are known (see, for example, H. C. Ansel and N. G. Popovish, Pharmaceutical Dosage Forms and Drug Delivery Systems, 5th ed., Lea and Febiger (1990)). Dosage levels and requirements are well-recognized in the art and may be selected by those of ordinary skill in the art from available methods and techniques suitable for a particular patient. In some embodiments, dosage levels range from 1-300 µg/dose for a 70 kg patient. Although one dose may be sufficient, up to 5 doses at intervals of months may be given. For oral doses, up to 20 mg/dose may be required. Reference in this regard may also be made to US provisional application no. 60/339,249. As the skilled artisan will appreciate, lower or higher doses may be required depending on particular factors. For instance, specific doses and treatment regimens will depend on factors such as the patient's general health profile, the severity and course of the patient's disorder or disposition thereto, and the judgment of the treating physician.

The present invention is also directed to the use of the self-assembling glycosylated capsid polypeptide as described herein or the composition described herein the manufacture of a medicament, optionally a vaccine.

In another aspect the present invention is directed to a method of treatment, optionally a method of vaccinating a patient, optionally a mammalian patient, optionally a human patient, the method comprising the step of administering a therapeutically effective amount of a polypeptide or composition of the present invention to a patient in need of such treatment, e.g. a patient suffering from a microbiological, optionally a bacterial infection.

In the following, an overview of the CPS names used in the present invention is provided.

| **Overview of CPS names** | |
|---|---|
| *Escherichia coli* | |
| CPS name | Structure. Ref. |
| The CPS-specific biosynthesis genes of *E. coli* are limited to those immediately flanked by *kpsMF* and *kpsFEDUCS* as defined in (Roberts 1996). | |
| K1 | (Jann and Jann 1990) |
| K2a/K2b | (Jann and Jann 1990) |
| K3 | (Jann and Jann 1990) |
| K4 | (Jann and Jann 1990) |
| K5 | (Jann and Jann 1990) |
| K6 | (Jann and Jann 1990) |
| K7/K56 | (Jann and Jann 1990) |
| K10 | (Sieberth, Jann, and Jann 1993) |
| K11 | (Jann and Jann 1990) |
| K12/K82 | (Jann and Jann 1990) |
| K13 | (Jann and Jann 1990) |
| K14 | (Jann and Jann 1990) |
| K15 | (Jann and Jann 1990) |
| K16 | (Jann and Jann 1990) |
| K18 | (Jann and Jann 1990) |
| K19 | (Jann and Jann 1990) |
| K20 | (Jann and Jann 1990) |
| K22 | (Jann and Jann 1990) |
| K23 | (Jann and Jann 1990) |
| K24 | (Jann and Jann 1990) |
| K51 | (Jann and Jann 1990) |
| K52 | (Jann and Jann 1990) |
| K53 | (Jann and Jann 1990) |
| K54 | (Jann and Jann 1990) |
| K74 | (Jann and Jann 1990) |
| K92 | (Jann and Jann 1990) |
| K93 | (Jann and Jann 1990) |
| K95 | (Jann and Jann 1990) |
| K96 | (Jann, Kochanowski, and Jann 1994) |
| K97 | (Jann and Jann 1990) |
| K98 | (Hahne, Jann, and Jann 1991) |
| K100 | (Jann and Jann 1990) |

| *Neisseria meningitidis* | |
|---|---|
| The CPS-specific biosynthesis genes of_*N*. *meningitidis* are limited to those flanked by the capsule transport genes *ctrABCD* and the LPS biosynthesis gene *galE* as defined in (Harrison et al. 2013). | |
| CPS name | Structure Ref. |
| A | (Harrison et al. 2013) |
| B | (Harrison et al. 2013) |
| C | (Harrison et al. 2013) |
| E | (Bhattacharjee, Jennings, and Kenny 1978) |
| H | (Beynon, Richards, and Perry 1992) |
| I | (Michon et al. 1985) |
| K | (Kaaden et al. 1985) |
| L | (Litschko et al. 2018) |
| W | (Bhattacharjee et al. 1976) |
| X | (Oldrini et al. 2018) |
| Y | (Bhattacharjee et al. 1976) |
| Z | (Jennings, Rosell, and Kenny 1979) |

| *Pasturella multocida* | |
|---|---|
| The CPS-specific biosynthesis genes of *P*. *multocida* are limited to those directly flanked by *hexDCBA* and *phyAB* as defined in (Watt et al. 2003). | |
| CPS name | Structure Ref. |
| A | (DeAngelis et al. 2002) |
| D | (DeAngelis et al. 2002) |
| F | (DeAngelis et al. 2002) |

| *Haemophilus influenzae* | |
|---|---|
| The CPS-specific biosynthesis genes of *H*. *influenzae* are limited to those in the genomic region flanked by *bexDCAB* and *hcsAB* as defined in (Giufre et al. 2010). | |
| CPS name | Structure Ref. |
| a | (Egan et al. 1982) |
| b | (Egan et al. 1982) |
| c | (Litschko et al. 2018) |
| d | (Jin et al. 2007) |
| e | (Jin et al. 2007) |
| e' | (Jin et al. 2007) |
| f | (Egan et al. 1982) |

| *Campylobacter jejuni* | |
|---|---|
| The CPS-specific biosynthesis genes of *C*. *jejuni* are limited to those in the genomic region flanked by *kpsF and kpsC as* defined in (Poly et al. 2015). | |
| CPS name | Structure Ref. |
| HS1/44 | (Karlyshev et al. 2005) |
| HS2 | (Karlyshev et al. 2005) |
| HS3 | (Aspinall et al. 1995) |
| HS4 | (Chen et al. 2008) |
| HS5 | capsule gene cluster defined in (Poly et al. 2015) |
| HS6 | (Muldoon et al. 2002) |
| HS8 | capsule gene cluster defined in (Liang et al. 2016) |
| HS9 | capsule gene cluster defined in (Liang et al. 2016) |
| HS10 | capsule gene cluster defined in (Liang et al. 2016) |
| HS11 | capsule gene cluster defined in (Poly et al. 2015) |
| HS12 | capsule gene cluster defined in (Liang et al. 2016) |
| HS15 | (Bertolo et al. 2013) |
| HS18 | capsule gene cluster defined in (Poly et al. 2015) |
| HS19 | (Karlyshev et al. 2005) |
| HS21 | capsule gene cluster defined in (Liang et al. 2016) |
| HS22 | capsule gene cluster defined in (Poly et al. 2015) |
| HS23/HS36 | (Karlyshev et al. 2005) |
| HS27 | capsule gene cluster defined in (Poly et al. 2015) |
| HS29 | capsule gene cluster defined in (Poly et al. 2015) |
| HS32 | capsule gene cluster defined in (Liang et al. 2016) |
| HS33 | capsule gene cluster defined in (Poly et al. 2015) |
| HS35 | capsule gene cluster defined in (Poly et al. 2015) |
| HS37 | capsule gene cluster defined in (Liang et al. 2016) |
| HS38 | capsule gene cluster defined in (Poly et al. 2015) |
| HS40 | capsule gene cluster defined in (Poly et al. 2015) |
| HS41 | (Karlyshev et al. 2005) |
| HS42 | capsule gene cluster defined in (Liang et al. 2016) |
| HS45 | capsule gene cluster defined in (Poly et al. 2015) |
| HS52 | capsule gene cluster defined in (Poly et al. 2015) |
| HS53 | (Gilbert et al. 2007) |
| HS55 | capsule gene cluster defined in (Poly et al. 2015) |
| HS57 | capsule gene cluster defined in (Poly et al. 2015) |
| HS58 | capsule gene cluster defined in (Poly et al. 2015) |
| HS60 | capsule gene cluster defined in (Poly et al. 2015) |
| HS62 | capsule gene cluster defined in (Poly et al. 2015) |
| HS63 | capsule gene cluster defined in (Poly et al. 2015) |

| *Actinobacillus pleuropneumoniae* | |
|---|---|
| The CPS-specific biosynthesis genes of A. *pleuropneumoniae* are limited to those in the genomic region flanked by *cpsDCBA* and *ydeN* as defined in_(Bossé et al. 2018). | |
| CPS name | Structure Ref. |
| 1 | (Litschko et al. 2018) |
| 2 | (E. Altman, Brisson, and Perry 1987) |
| 3 | (Eleonora Altman, Brisson, and Perry 1987) |
| 4 | (Eleonora Altman, Brisson, and Perry 1988) |
| 5 | capsule gene cluster as defined in (Bossé et al. 2018) |
| 6 | capsule gene cluster as defined in (Bossé et al. 2018) |
| 7 | (Beynon, Perry, and Richards 1991) |
| 8 | capsule gene cluster as defined in (Bossé et al. 2018) |
| 9 | (Beynon, Richards, and Perry 1992) |
| 10 | capsule gene cluster as defined in (Bossé et al. 2018) |
| 11 | capsule gene cluster as defined in (Bossé et al. 2018) |
| 12 | (Litschko et al. 2018) |
| 13 | capsule gene cluster as defined in (Bossé et al. 2018) |
| 14 | capsule gene cluster as defined in (Bossé et al. 2018) |
| 15 | capsule gene cluster as defined in (Bossé et al. 2018) |
| 16 | capsule gene cluster as defined in (Bossé et al. 2018) |
| 17 | capsule gene cluster as defined in (Bossé et al. 2018) |
| 18 | capsule gene cluster as defined in (Bossé et al. 2018) |

| *Bibersteinia trehalosi* | |
|---|---|
| CPS name | Structure Ref. |
| T3 | (Richards and Leitch 1990) |
| T4 | (Adlam et al. 1985) |
| T15 | (Beynon, Richards, and Perry |
| 1992) | |
| *Mannheimia haemolytica* | |

| *Mannheimia haemolytica* | |
|---|---|
| The CPS-specific biosynthesis genes of *M. haemolytica* are limited to those in the genomic region flanked by *wza* and *wbrA* as defined in (Lo et al. 2001). | |
| CPS name | Structure Ref. |
| A1 | (Adlam et al. 1985) |
| A2 | (Robbins et al. 2011) |

| *Cronobacter sakazakii* | |
|---|---|
| The CPS-specific biosynthesis genes of C. *sakazakii* are limited to those in the genomic region flanked by *kpsEDCS* and *kpsMT* as defined in (Ogrodzki and Forsythe 2015) | |
| CPS name | Structure Ref. |
| K1 | capsule gene cluster as defined in (Ogrodzki and Forsythe 2015) |
| K2 | capsule gene cluster as defined in (Ogrodzki and Forsythe 2015) |

### References

Adlam, C., J. M. Knights, A. Mugridge, J. C. Lindon, and J. M. Williams. 1985. Microbiology 131 (2): 387-94.
Altman, E., J. R. Brisson, and M. B. Perry. 1987. Biochemistry and Cell Biology = Biochimie Et Biologie Cellulaire 65 (5): 414-22.
Altman, Eleonora, Jean-Robert Brisson, and Malcolm B. Perry. 1987. Biochemistry and Cell Biology 65 (11): 960-67.
Altman, Eleonora et al. 1988. "Structural Studies of the Capsular Polysaccharide from Actinobacillus (Haemophilus) Pleuropneumoniae Serotype 4." Biochemistry and Cell Biology 66 (9): 998-1004.
Aspinall, G. O., C. M. Lynch, H. Pang, R. T. Shaver, and A. P. Moran. 1995. European Journal of Biochemistry 231 (3): 570-78.
Bertolo, Lisa, Cheryl P. Ewing, Alexander Maue, Frederic Poly, Patricia Guerry, and Mario A. Monteiro. 2013. Carbohydrate Research 366 (January): 45-49.
Beynon, L. M., M. B. Perry, and J. C. Richards. 1991. Carbohydrate Research 209: 211-23.
Beynon, L. M., J. C. Richards, and M. B. Perry. 1992. European Journal of Biochemistry 210 (1): 119-24.
Bhattacharjee, Apurba K., Harold J. Jennings, and C. Paul Kenny. 1978. Biochemistry 17 (4): 645-51.
Bhattacharjee, Apurba K., Harold J. Jennings, C. Paul Kenny, Adèle Martin, and Ian C. P. Smith. 1976. Canadian Journal of Biochemistry 54 (1): 1-8.
Bossé, Janine T., Yanwen Li, Roberto Fernandez Crespo, Sonia Lacouture, Marcelo Gottschalk, Rita Sárközi, László Fodor, et al. 2018. Veterinary Microbiology 220 (July): 83-89.
Chen, Yu-Han, Frédéric Poly, Zbigniew Pakulski, Patricia Guerry, and Mario A. Monteiro. 2008. Carbohydrate Research 343 (6): 1034-40.
DeAngelis, Paul L., Nur Sibel Gunay, Toshihiko Toida, Wen-jun Mao, and Robert J. Linhardt. 2002. Carbohydrate Research 337 (17): 1547-52.
Egan, William, Rachel Schneerson, Kathleen E. Werner, and Gerald Zon. 1982. Journal of the American Chemical Society 104 (10): 2898-2910.
Gilbert, Michel, Robert E. Mandrell, Craig T. Parker, Jianjun Li, and Evgeny Vinogradov. 2007. Chembiochem: A European Journal of Chemical Biology 8 (6): 625-31.
Giufre, M., R. Cardines, P. Mastrantonio, and M. Cerquetti. 2010. Journal of Clinical Microbiology 48 (4): 1404-7.
Hahne, M., B. Jann, and K. Jann. 1991. Carbohydrate Research 222: 245-53.
Harrison, Odile B., Heike Claus, Ying Jiang, Julia S. Bennett, Holly B. Bratcher, Keith A. Jolley, Craig Corton, et al. 2013. Emerging Infectious Diseases 19 (4): 566-73.
Jann, B., and K. Jann. 1990. Current Topics in Microbiology and Immunology 150: 19-42.
Jann, B., H. Kochanowski, and K. Jann. 1994. Carbohydrate Research 253: 323-27.
Jennings, Harold J., Karl-Gunnar Rosell, and C. Paul Kenny. 1979. Canadian Journal of Chemistry 57 (22): 2902-7.
Jin, Zhigang, Sandra Romero-Steiner, George M. Carlone, John B. Robbins, and Rachel Schneerson. 2007. Infection and Immunity 75 (6): 2650-54.
Kaaden, Arie, Gerrit J. Gerwig, Johannis P. Kamerling, Johannes F. G. Vliegenthart, and Rudy H. Tiesjema. 1985. European Journal of Biochemistry 152 (3): 663-68.
Karlyshev, Andrey V., Olivia L. Champion, Carol Churcher, Jean-Robert Brisson, Harold C. Jarrell, Michel Gilbert, Denis Brochu, et al. 2005. Molecular Microbiology 55 (1): 90-103.
Liang, Hao, Aiyu Zhang, Yixin Gu, Yuanhai You, Jianzhong Zhang, and Maojun Zhang. 2016. PloS One 11 (10): e0165159.
Litschko, Christa, Davide Oldrini, Insa Budde, Monika Berger, Jochen Meens, Rita Gerardy-Schahn, Francesco Berti, Mario Schubert, and Timm Fiebig. 2018. MBio 9 (3).
Lo, R. Y. C., L. J. McKerral, T. L. Hills, and M. Kostrzynska. 2001. Infection and Immunity 69 (7): 4458-64.
Michon, Francis, Jean Robert Brisson, Rene Roy, Fraser E. Ashton, and Harold J. Jennings. 1985. Biochemistry 24 (20): 5592-98.
Muldoon, Jimmy, Alexander S. Shashkov, Anthony P. Moran, John A. Ferris, Sof'ya N. Senchenkova, and Angela V. Savage. 2002. Carbohydrate Research 337 (21-23): 2223-29.
Ogrodzki, P., and S. Forsythe. 2015. BMC Genomics 16 (1).
Oldrini, Davide, Timm Fiebig, Maria Rosaria Romano, Daniela Proietti, Monika Berger, Marta Tontini, Riccardo De Ricco, et al. 2018. ACS Chemical Biology 13 (4): 984-94.
Poly, Frédéric, Oralak Serichantalergs, Janelle Kuroiwa, Piyarat Pootong, Carl Mason, Patricia Guerry, and Craig T. Parker. 2015. Edited by Mikael Skurnik. PLOS ONE 10 (12): e0144349.
Richards, James C., and Robert A. Leitch. 1990. Canadian Journal of Chemistry 68 (9): 1574-84.
Robbins, John B., Rachel Schneerson, Guilin Xie, Lars Å Hanson, Lars Åke-Hanson, and Mark A. Miller. 2011. Proceedings of the National Academy of Sciences of the United States of America 108 (44): 17871-75.
Roberts, I. S. 1996. Annual Review of Microbiology 50: 285-315.
Sieberth, V., B. Jann, and K. Jann. 1993. Carbohydrate Research 246 (August): 219-28.
Watt, James M, Ed Swiatlo, Mary M Wade, and Franklin R Champlin. 2003. FEMS Microbiology Letters 225 (1): 9-14.

The following Figures and Examples serve to illustrate the invention and are not intended to limit the scope of the invention as described in the appended claims.

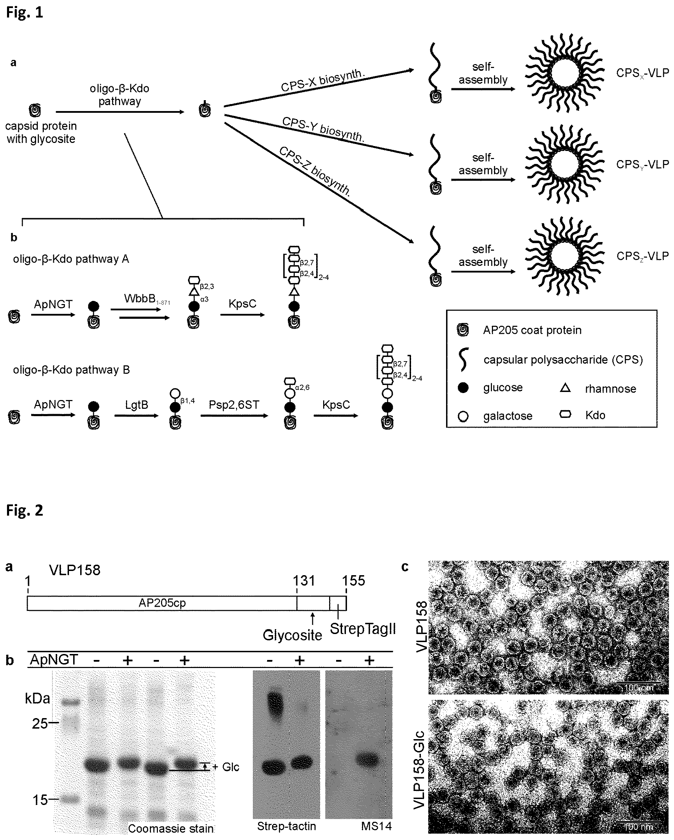

**Figure 1****:** shows the biosynthesis and structure of glycosylated self-assembling capsid proteins of the present invention (termed CPS-VLPs). (**a**) The biosynthesis of CPS-VLPs can occur in two phases. The first phase can be common to all CPS pathways, here a short oligosaccharide (oligo-β-Kdo) is synthesized onto the coat protein. The resulting structure can serve as a pathway hub, able to prime biosynthesis of diverse CPS structures. (**b**) Details of two exemplary biosynthetic pathways for protein-linked oligo-β-Kdo. WbbB₁₋₈₇₁ (SEQ ID NO: 3) and Psp2,6ST (SEQ ID NO: 7) transfer a single Kdo residue onto the protein-linked glucose or glucose-galactose, respectively. KpsC is a bifunctional β2,4-/β2,7-Kdo transferase which synthesizes the target oligo-β-Kdo structure (the primer for CPS biosynthesis).
**Figure 2****:** shows the glycosylation and assembly of a representative self-assembling glycosylated capsid polypeptide (VLP158, SEQ ID NO: 1). (**a**) Schematic of VLP158 capsid protein, a C-terminal extension including glycosylation site and affinity tag was genetically fused to the AP205 coat protein (AP205cp, SEQ ID NO: 1). (**b**) This construct was expressed in E. coli, in the presence and absence of the ApNGT (SEQ ID NO: 2) then affinity purified and analyzed by SDS-PAGE and immunoblot. A consistent increase in the apparent molecular weight following co-expression with the ApNGT suggests that the protein was modified with a single glucose (Glc) residue. Modification of VLP158 with Glc is confirmed by staining of the protein with an N-linked glucose reactive serum, MS14. (**c**) Transmission electron microscopy demonstrates that both the glycosylated and unmodified coat proteins assemble into regular capsids with a diameter of approximately 30 nm.

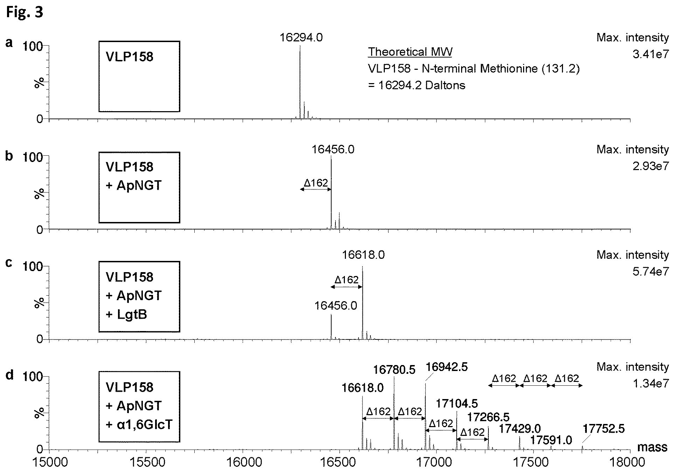

**Figure 3****:** shows the mass spectrometric analysis of glycosylated capsid proteins. The VLP158 capsid protein (SEQ ID NO: 1) was recombinantly expressed alone (**a**), in the presence of the ApNGT (SEQ ID NO: 2) (**b**), the ApNGT (SEQ ID NO: 2) and LgtB (SEQ ID NO: 5) (**c**), or the ApNGT (SEQ ID NO: 2) and the polymerizing α1,6-glucosyltransferase (α1,6GlcT, SEQ ID NO: 6) (**d**). Following expression for 20 hours at 28°C in *E. coli,* cells were harvested and cytoplasmic material extracted. The modified capsids were purified by affinity chromatography and characterized by electrospray time-of-flight mass spectrometry. When expressed in the absence of ApNGT, the measured molecular weight (MW) corresponds to the expected mass of VLP158 with quantitative removal of the N-terminal methionine residue. Upon co-expression with the ApNGT, the protein is modified by 162 Daltons, indicating the quantitative addition of a single glucose residue. Upon addition of further glycosyltransferases (LgtB or the α1,6GlcT), the protein is modified by addition of further hexoses (162 Daltons) consistent with biosynthesis of the target structures.

### Example 1: Glycosylation of the AP205 virus-like particle (AP205-VLP)

A capsid, or virus-like particle (VLP), is formed upon recombinant expression of the coat protein of the AP205 bacteriophage (AP205cp). This AP205-VLP is a well-established scaffold for presentation of vaccine antigens (A.C. Tissot et al. 2010, PLoS ONE, e9809; K.D. Brune et al. 2016, Sci. Rep., vol. 6, no. 1; M. Shishovs et al. 2016, J. Mol. Biol., 427-4279). It consists of 180 copies of the AP205cp assembled into a spherical capsid. The N- and C-termini of the coat protein are exposed on the surface of the assembled particle. To generate a capsid that can be N-glycosylated, a short polypeptide tag including a glycosylation sequon (Asn-x-Ser/Thr) was fused genetically at the C-terminus of the AP205cp, the engineered capsid protein is referred to as "VLP158" (SEQ ID NO: 1, Figure 2a). To test cytoplasmic glycosylation, this protein was co-expressed together with the N-glycosyltransferase of *Actinobacillus pleuropneumoniae* (ApNGT, SEQ ID NO: 2) in the cytoplasm of *E. coli.* The recombinant capsids were extracted from the cytoplasm of the expression host, affinity purified using strep-tactin beads, then characterized by SDS-PAGE, immune-blotting, mass spectrometry, and transmission electron microscopy. The observations from SDS-PAGE, immnoblotting and mass spectrometry indicated efficient addition of glucose to the coat protein in the presence of the ApNGT (Figure 2b, 3a & 3b). Transmission electron microscopy images were consistent with the assembly of spherical particles with a diameter of approximately 30 nm both with and without the N-linked glucose (Figure 2c). It was further demonstrated that the glucose can be extended by two different glycosyltransferases, the galactosyltransferase LgtB (SEQ ID NO: 5) of *Neisseria meningitidis* and the polymerizing α1,6-glucosyltransferase (SEQ ID NO: 6) of *Actinobacillus pleuropneumoniae,* (Figure 3c & 3d). The resulting capsids were modified with lactose or an α1,6-linked glucose oligomer, respectively.

## Claims

1. A self-assembling glycosylated capsid polypeptide, wherein the capsid polypeptide comprises at least one 3-deoxy-D-*manno*-oct-2-ulosonic acid (Kdo)-glycosylation.

2. The capsid polypeptide according to claim 1, wherein the capsid polypeptide comprises a glycosylation pattern selected from the group consisting of
(7) βKdo-(2,3)αRha-(1,3)-βGlc-(1,N)- or βKdo-(2,3)-αRha-(1,3)-βGlcNAc-(1,O)-;
(8) αKdo-(2,6)-βGal-(1,4)-βGlc-(1,N)- or αKdo-(2,6)-βGal-(1,4)-βGlcNAc-(1,O)-;
(9) [βKdo-(2,4)]ₘ-[βKdo-(2,7)-βKdo-(2,4)]ₙ-βKdo-(2,3)-αRha-(1,3)-βGlc-(1,N)- or [βKdo-(2,4)]ₘ-[βKdo-(2,7)-βKdo-(2,4)]ₙ-βKdo-(2,3)-αRha-(1,3)-βGlcNAc-(1,O)-;
(10) [βKdo-(2,4)]ₘ-[βKdo-(2,7)-βKdo-(2,4)]ₙ-αKdo-(2,6)-βGal-(1,4)-βGlc-(1,N)- or [βKdo-(2,4)]ₘ-[βKdo-(2,7)-βKdo-(2,4)]ₙ-αKdo-(2,6)-βGal-(1,4)-βGlcNAc-(1,O)-;
(11) [βKdo-(2,7)]ₘ-[βKdo-(2,4)-βKdo-(2,7)]ₙ-βKdo-(2,3)-αRha-(1,3)-βGlc-(1,N)- or βKdo-(2,7)]ₘ-[βKdo-(2,4)-βKdo-(2,7)]ₙ-βKdo-(2,3)-αRha-(1,3)-βGlcNAc-(1,O)-; and
(12) [βKdo-(2,7)]ₘ-[βKdo-(2,4)-βKdo-(2,7)]ₙ-αKdo-(2,6)-βGal-(1,4)-βGlc-(1,N)- or [βKdo-(2,7)]ₘ-[βKdo-(2,4)-βKdo-(2,7)]ₙ-αKdo-(2,6)-βGal-(1,4)-βGlcNAc-(1,O)-;
wherein m is 0 or 1 and n is an integer from 2 to 4 and wherein the glycosylation pattern is connected to the capsid polypeptide at the reducing end.

3. The capsid polypeptide according to claim 1 or 2, wherein the at least one glycosylation is an N- or O-glycosylation of the capsid polypeptide, optionally
(1) an N-glycosylation at a glycosylation site Asn-X-Ser/Thr of the capsid polypeptide, wherein X is not Pro, or
(2) an O-glycosylation at a glycosylation site M-N-O-P, wherein
M is selected from the group consisting of Pro, Thr and Arg,
N is selected from the group consisting of Val, Thr and Arg,
O is selected from the group consisting of Ser and Thr, and
P is selected from the group consisting of Arg, Leu, Val, Ser and Thr (SEQ ID NO: 136).

4. The capsid polypeptide according to any of claims 1 to 3, wherein the glycosylation pattern is selected from claim 1 or 2 and extended by at least one further glycoside, optionally capsular polysaccharides (CPS), optionally a CPS selected from the group consisting of:
(a) *E. coli* CPS selected from the group consisting of CPS K1, K2a/K2b, K3 to K6, K7/K56, K10, K11, K12/82, K13 to K16, K18 to K20, K22 to K24, K51 to K54, K74, K92, K93, K95 to K98 and K100;
(b) *Neisseria meningitidis* CPS selected from the group consisting of A to C, E, H, I, K, L, W, X, Y and Z;
(c) *Pasteurella multocida* CPS selected from the group consisting of CPS A, D and F;
(d) *Haemophilus influenzae* CPS selected from the group consisting of CPS a to d, e, e', and f;
(e) *Campylobacter jejuni* CPS selected from the group consisting of CPS HS1/44, HS2 to HS6, HS8 to HS12, HS15, HS18, HS19, HS21, HS22, HS23/36, HS27, HS29, HS32, HS33, HS35, HS37, HS38, HS40 to HS42, HS45, HS52, HS53, HS55, HS57, HS58, HS60, HS62, and HS63;
(f) *Actinobacillus pleuropneumoniae* CPS selected from the group consisting of CPS 1 to 18;
(g) *Bibersteinia trehalosi* CPS selected from the group consisting of CPS T3, T4 and T15;
(h) *Mannheimia haemolytica* CPS selected from the group consisting of A1 and A2; and
(i) *Cronobacter sakazakii* CPS selected from the group consisting of K1 and K2.

5. The capsid polypeptide according to any of claims 1 to 4, wherein the self-assembling capsid polypeptide is selected from the group consisting of:
(a) a capsid polypeptide comprising or consisting of an amino acid sequence selected from the group consisting of SEQ ID NOs: 1 and 91 to 108;
(b) a capsid polypeptide comprising an amino acid sequence having an amino acid sequence identity of at least 70% or 80%, optionally at least 90% or 95%, optionally at least 98% with an amino acid sequence selected from the group consisting of SEQ ID NOs: 1 and 91 to 108; and
(c) a capsid polypeptide comprising a functional derivative and/or functional fragment of (a) and/or (b).

6. The capsid polypeptide according to any of claims 1 to 5, wherein
(a) the self-assembling capsid polypeptide is a capsid polypeptide comprising an amino acid sequence according to SEQ ID NO: 1,
(b) the glycosylation pattern is [βKdo-(2,4)]ₘ-[βKdo-(2,7)-βKdo-(2,4)]ₙ-βKdo-(2,3)-αRha-(1,3)-βGlc-(1,N) or [βKdo-(2,7)]ₘ-[βKdo-(2,4)-βKdo-(2,7)]ₙ-βKdo-(2,3)-αRha-(1,3)-βGlc-(1,N), wherein m is 0 or 1 and n is an integer from 2 to 4, optionally 2, and
(c) the at least one further glycoside is absent or is a CPS selected from the group consisting of:
*Neisseria meningitidis* serogroup A, C, L, W-135, X, and Y;
*Haemophilus influenzae* type b and c;
*Pasteurella multocida* serogroup A;
*Actinobacillus pleuropneumoniae* serotype 6 and 8;
*Mannheimia haemolytica* serotype A1;
*Escherichia coli* serotype K2, K16, K20, K22, K23 and K100;
*Campylobacter jejuni* serotype HS1/44 HS2, HS3, HS4, HS5, HS8; and
*Cronobacter sakazakii* serotype K1 and K2.

7. A method for producing self-assembling capsid polypeptides according to any of claims 1 to 6, comprising the steps of:
(i) providing at least one bacterial host cell expressing in its cytoplasm
(1) at least one self-assembling capsid polypeptide comprising at least one glycosylation site, optionally an N- or O-glycosylation site on the outer surface of the assembled capsid;
(2) at least one N- or O- glycosyl transferase enzyme (NGT or OGT) for glycosylating the at least one glycosylation site of the capsid polypeptide; and
(3) at least one glycosylation enzyme for glycosylating the glycosyl residue(s) of the at least one glycosylation site of the capsid polypeptide of step (2),
(ii) culturing the host cell under physiological conditions so that
(1) self-assembling capsid polypeptides are expressed, and
(2) the at least one glycosyl transferase enzyme (NGT or OGT) glycosylates the at least one glycosylation site of the capsid polypeptide, and
(3) the at least one glycosylation enzyme glycosylates the glycosyl residue(s) of the at least one glycosylation site of the capsid polypeptide;
(iii) optionally isolating the glycosylated capsid polypeptide(s) from the host cell.

8. The method according to claim 7 featuring two or more bacterial host cells, each of them expressing at least one of (a) the capsid polypeptide, (b) the N- or O- glycosyl transferase enzyme (NGT or OGT) and/or (c) the glycosylation enzyme for glycosylating the glycosyl residue(s) of the at least one glycosylation site of the capsid polypeptide.

9. The method according to claim 7 or 8, wherein the bacterial host cell(s) is a gram-negative or gram-positive host cell, optionally selected from the group consisting of *Escherichia* species, *Shigella* species, *Neisseria* species, *Haemophilus* species, *Pasteurella* species, *Actinobacillus* species, *Klebsiella* species, *Xhantomonas* species, *Salmonella* species, *Yersinia* species, *Lactococcus* species, *Lactobacillus* species, *Pseudomonas* species, *Corynebacterium* species, *Streptomyces* species, *Streptococcus* species, *Staphylococcus* species, *Bacillus* species, and *Clostridium* species.

10. The method according to any of claims 7 to 9, wherein the bacterial host cell(s) is a cell-free system derived from a bacterial cell by lysis.

11. The method according to any of claims 7 to 10, wherein
(I) the self-assembling capsid polypeptide is selected from the group consisting of:
(a) a capsid polypeptide comprising or consisting of an amino acid sequence selected from the group consisting of SEQ ID NO: 1 and 91 to 108;
(b) a capsid polypeptide comprising an amino acid sequence having an amino acid sequence identity of at least 70% or 80%, optionally at least 90% or 95%, optionally at least 98% with an amino acid sequence selected from the group consisting of SEQ ID NOs: 1, and 91 to 108; and
(c) a capsid polypeptide comprising a functional derivative and/or functional fragment of (a) and/or (b);
and/or
(II) the at least one glycosyl transferase enzyme (NGT or OGT) is selected from the group consisting of:
(a) a glycosyl transferase enzyme comprising or consisting of an amino acid sequence selected from the group consisting of SEQ ID NO: 2 and SEQ ID NOs: 109 to 133,
(b) a glycosyl transferase enzyme comprising an amino acid sequence having an amino acid sequence identity of at least 70% or 80%, optionally at least 90% or 95%, optionally at least 98% with an amino acid sequence selected from the group consisting of SEQ ID NOs: 2 and 109 to 133; and
(c) a glycosyl transferase enzyme comprising a functional derivative and/or functional fragment of (a) and/or (b).

12. The method according to any of claims 7 to 11, wherein the at least one glycosylation enzyme for glycosylating the glycosyl residue(s) of the at least one glycosylation site of the capsid polypeptide according to steps (i)(3) and (ii)(3) of claim 7 is selected from the group consisting of
(3a) a WbbB homolog comprising or consisting of an amino acid sequence selected from the group consisting of SEQ ID NOs: 3 and 29 to 90;
(3b) a bacterial polymerizing 3-deoxy-β-D-manno-oct-2-ulopyranosonic acid (β-Kdo) glycosyltransferase comprising or consisting of an amino acid sequence selected from the group consisting of SEQ ID NOs: 4 and 8 to 18;
(3c) an LgtB glycosylation enzyme comprising or consisting of an amino acid sequence according to SEQ ID NO: 5;
(3d) a Psp2,6ST homolog comprising or consisting of an amino acid sequence selected from the group consisting of SEQ ID NOs: 7 and 19 to 28; and
(3e) an alpha1,6GlcT homolog comprising or consisting of an amino acid sequence selected from the group consisting of SEQ ID NOs: 6, 134 and 135;
(3f) a glycosylation enzyme comprising an amino acid sequence having an amino acid sequence identity of at least 70% or 80%, optionally at least 90% or 95%, optionally at least 98% with an amino acid sequence selected from the group consisting of SEQ ID NOs: 3 to 90, 134 and 135; and
(3g) a glycosylation enzyme comprising a functional derivative and/or functional fragment of any of (3a) to (3f);
wherein the LgtB glycosylation enzyme of (3c), (3f) or (3g) or the alpha1,6GlcT homolog of (3e), (3f) or (3g) requires the presence of at least one further glycosylation enzyme selected from the WbbB homolog of (3a), (3f) or (3g), the bacterial polymerizing 3-deoxy-β-D-manno-oct-2-ulopyranosonic acid (β-Kdo) glycosyltransferase of (3b), (3f) or (3g) and/or the Psp2,6ST homolog of (3d), (3f) or (3g).

13. Method according to any of claims 7 to 12, wherein the at least one bacterial host cell further expresses enzyme(s) or enzyme complex(es) for extending the glycosylation of the capsid polypeptide with at least one capsular polysaccharide (CPS), optionally a CPS according to claim 4, optionally enzymes selected from the group consisting of serotype specific CPS biosynthesis enzymes.

14. A composition, optionally a medical composition comprising a self-assembling capsid polypeptide according to any of claims 1 to 6, optionally comprising immunogenic compounds, optionally mRNA.

15. The self-assembling glycosylated capsid polypeptide according to any of claims 1 to 6 or the composition according to claim 14 for use in medical treatment, optionally for use as a vaccine.
